(19) Europäisches Patentamt
European Patent Office
Office européen des brevets

(11) Veröffentlichungsnummer: 0 664 283 A1

# (12) EUROPÄISCHE PATENTANMELDUNG

(21) Anmeldenummer: 95100595.8

(22) Anmeldetag: 18.01.95

(51) Int. Cl.[6]: C07C 233/42, C07C 233/43, C07C 233/44, C07D 309/08, C07D 309/28, C07C 327/38, A01N 37/22

(30) Priorität: 25.01.94 DE 4402056

(43) Veröffentlichungstag der Anmeldung: 26.07.95 Patentblatt 95/30

(84) Benannte Vertragsstaaten: AT BE CH DE DK ES FR GB GR IE IT LI NL PT SE

(71) Anmelder: BASF Aktiengesellschaft
Carl-Bosch-Strasse 38
D-67063 Ludwigshafen (DE)

(72) Erfinder: Eicken, Dr. Karl
Am Hüttenwingert 12
D-67157 Wachenheim (DE)
Erfinder: Wagner, Dr. Oliver
Siemensstrasse 1
D-66450 Bexbach (DE)
Erfinder: Rang, Dr. Harald
Ziegeleistrasse 76
D-67122 Altrip (DE)
Erfinder: Ammermann, Dr. Eberhard
Von-Gagern-Strasse 2
D-64646 Heppenheim (DE)
Erfinder: Lorenz, Dr. Gisela
Erlenweg 13
D-67434 Neustadt (DE)

## (54) P-Phenylendiamin-Derivate und diese enthaltende fungizide Mittel.

(57) p-Phenylendiamin-Derivate I

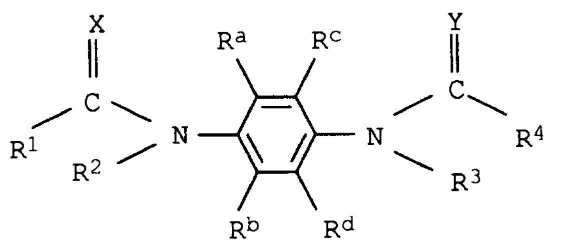

I

| | |
|---|---|
| $R^1$ = | geg. subst. $C_3$-$C_8$-Alkyl, $C_2$-$C_8$-Alkenyl, $C_2$-$C_8$-Alkinyl, $C_3$-$C_6$ Cycloalkyl, $C_5$-$C_8$-Bicycloalkyl, $C_5$-$C_8$-Bicycloalkenyl, Dihydropyranyl oder Tetrahydropyranyl; |
| $R^2$ = | H oder gemeinsam mit $R^1$-CX-N- einen geg. subst. Azetidin-2-on Ring; |
| $R^3$ = | $C_1$-$C_6$-Alkyl, $C_2$-$C_6$-Alkenyl, $C_3$-$C_6$-Alkinyl, $C_3$-$C_6$-Cycloalkyl, $C_3$-$C_6$-Cycloalkyl-$C_1$-$C_3$-alkyl, $C_1$-$C_4$-Alkoxy-$C_2$-$C_4$-alkyl, $C_3$-$C_4$-Alkenyloxy-$C_2$-$C_4$-alkyl; |
| $R^4$ = | H oder $C_1$-$C_6$-Alkyl, $C_2$-$C_6$-Alkenyl, $C_3$-$C_6$-Alkinyl, $C_3$-$C_6$-Cycloalkyl, durch $C_1$-$C_3$-Alkyl substituiertes $C_3$-$C_6$-Cycloalkyl, wobei diese Gruppen jeweils 1 bis 3 Halogenatome tragen können; |
| $R^a$, $R^b$ = | H, F; $R^c$, $R^d$ = H, CN, $NO_2$, Halogen, $C_1$-$C_4$-Alkyl, $C_1$-$C_4$-Alkoxy, $C_1$-$C_4$-Halogenalkyl, ($C_1$-$C_4$-Alkoxy)carbonyl, $C_1$-$C_4$-Alkylcarbonyl oder CHO, mit der Maßgabe, daß $R^c$ und $R^d$ nicht gleichzeitig H sein können; |
| X, Y = | O oder S. |

EP 0 664 283 A1

Die vorliegende Erfindung betrifft p-Phenylendiamin-Derivate der Formel I

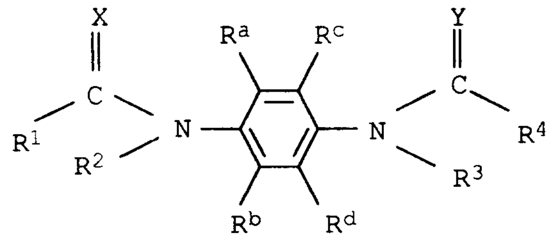

I

in der die Substituenten folgende Bedeutung haben:

- $R^1$ $C_3$-$C_8$-Alkyl, $C_2$-$C_8$-Alkenyl, $C_2$-$C_8$-Alkinyl, $C_3$-$C_6$-Cycloalkyl, $C_5$-$C_8$-Bicycloalkyl, $C_5$-$C_8$-Bicycloalkenyl, Dihydropyranyl oder Tetrahydropyranyl, wobei diese Gruppen durch $C_1$-$C_4$-Alkyl, Halogen, $C_1$-$C_4$-Alkoxy, $C_1$-$C_4$-Alkylthio, Cyano, Isocyano, ($C_1$-$C_4$-Alkoxy)carbonyl, ($C_1$-$C_4$-Alkyl)carbonyl, Azido oder Nitro substituiert sein können;
- $R^2$ Wasserstoff oder gemeinsam mit dem $R^1$-CX-N- Teil einen Azetidin-2-on Ring, der gewünschtenfalls ein oder zwei $C_1$-$C_4$-Alkylreste tragen kann;
- $R^3$ $C_1$-$C_6$-Alkyl, $C_2$-$C_6$-Alkenyl, $C_3$-$C_6$-Alkinyl, $C_3$-$C_6$-Cycloalkyl, $C_3$-$C_6$-Cycloalkyl-$C_1$-$C_3$-alkyl, $C_1$-$C_4$-Alkoxy-$C_2$-$C_4$-alkyl, $C_3$-$C_4$-Alkenyloxy-$C_2$-$C_4$-alkyl;
- $R^4$ Wasserstoff oder $C_1$-$C_6$-Alkyl, $C_2$-$C_6$-Alkenyl, $C_3$-$C_6$-Alkinyl, $C_3$-$C_6$-Cycloalkyl, durch $C_1$-$C_3$-Alkyl substituiertes $C_3$-$C_6$-Cycloalkyl, wobei diese Gruppen jeweils 1 bis 3 Halogenatome tragen können.
- $R^a$, $R^b$ Wasserstoff oder Fluor;
- $R^c$, $R^d$ Wasserstoff, Cyano, Nitro, Halogen, $C_1$-$C_4$-Alkyl, $C_1$-$C_4$-Alkoxy, $C_1$-$C_4$-Halogenalkyl, ($C_1$-$C_4$-Alkoxy)carbonyl, $C_1$-$C_4$-Alkylcarbonyl oder Formyl, mit der Maßgabe, daß $R^c$ und $R^d$ nicht gleichzeitig Wasserstoff sein können;
- X, Y Sauerstoff oder Schwefel.

Außerdem betrifft die Erfindung Verfahren und neue Zwischenprodukte zur Herstellung dieser Verbindungen, ihre Verwendung als Fungizide, Fungizide Mittel, welche diese Verbindungen als wirksame Substanzen enthalten, sowie Verfahren zur Bekämpfung von Schadpilzen.

Unter die in der BE-A 863074 beschriebenen Verbindungen fallen auch Cycloalkylcarbonsäureanilide der Formel II

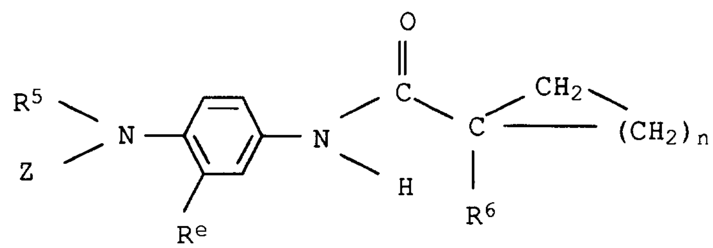

II,

wobei Z u. a. für Alkyl oder Cycloalkyl, $R^5$ u. a. für CO-Alkyl und $R^e$ u. a. für Halogen, Cyano, Nitro, Alkylcarbonyl oder Alkoxy stehen. Für diese Verbindungen, von denen jedoch kein einziger Vertreter aufgeführt ist, wird eine herbizide Wirkung angegeben.

Aufgabe der vorliegenden Erfindung war es, neue fungizid wirksame Verbindungen bereitzustellen.

Demgemäß wurden die eingangs definierten p-Phenylendiamin-Derivate I gefunden. Weiterhin wurden Verfahren zur Herstellung dieser Verbindungen, sie enthaltende Mittel und Verfahren zur Bekämpfung von Schadpilzen mit diesen Mitteln gefunden.

Außerdem wurden neue Zwischenprodukte der Formel III

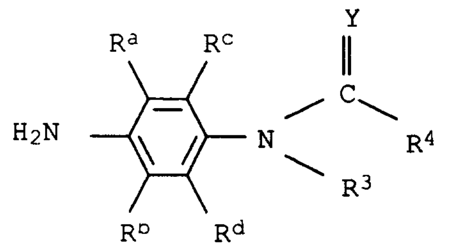

III

zur Herstellung der p-Phenylendiamin-Derivate I gefunden.

Die vorstehend für die Substituenten $R^1$ bis $R^4$ und $R^a$ bis $R^d$ genannten Bedeutungen Halogen, $C_1$-$C_4$-Alkyl, $C_1$-$C_6$-Alkyl, $C_3$-$C_8$-Alkyl,$C_1$-$C_4$-Halogenalkyl, $C_2$-$C_6$-Alkenyl, $C_2$-$C_8$-Alkenyl, $C_3$-$C_6$-Alkinyl, $C_2$-$C_8$-Alkinyl, $C_3$-$C_6$-Cycloalkyl, $C_5$-$C_8$-Bicycloalkyl, $C_5$-$C_8$-Bicycloalkenyl, $C_3$-$C_6$-Cycloalkyl-$C_1$-$C_3$-alkyl, ($C_1$-$C_4$-Alkyl)carbonyl, $C_1$-$C_4$-Alkoxy, $C_1$-$C_4$-Alkylthio, ($C_1$-$C_4$-Alkoxy)carbonyl, ($C_2$-$C_4$-Alkoxy)carbonyl, $C_1$-$C_4$-Alkoxy-$C_2$-$C_4$-alkyl und $C_3$-$C_4$-Alkenyloxy-$C_2$-$C_4$-alkyl stellen Sammelbegriffe für individuelle Aufzählungen der einzelnen Gruppenmitglieder dar. Sämtliche Kohlenstoffketten, also alle Alkyl-, Halogenalkyl-, Alkenyl-, Alkinyl-, Cycloalkyl-, Bicycloalkyl-, Bicycloalkenyl-, Alkoxy-, Alkylthio- und Alkenyloxy-Teile können geradkettig oder verzweigt sein. Mehrfach Halogen-substituierte Reste können gleiche oder verschiedene Halogenatome tragen.

Im einzelnen bedeuten beispielsweise

- Halogen: Fluor, Chlor, Brom und Jod;
- $C_1$-$C_4$-Alkyl: Methyl, Ethyl, n-Propyl, 1-Methylethyl, n-Butyl, 1-Methylpropyl, 2-Methylpropyl und 1,1-Dimethylethyl;
- $C_1$-$C_6$-Alkyl: $C_1$-$C_4$-Alkyl wie vorstehend genannt, und $C_5$-$C_6$-Alkyl wie n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
- $C_3$-$C_8$-Alkyl: n-Propyl, 1-Methylethyl, n-Butyl, 1-Methylpropyl, 2-Methylpropyl, 1,1-Dimethylethyl, $C_5$- und $C_6$-Alkyl wie vorstehend genannt, sowie z.B. n-Heptyl, 1-Methylhexyl, 1-Ethylpentyl, 2-Ethylpentyl, 1-Propylbutyl, n-Octyl, 1-Methylheptyl, 2-Methylheptyl, 1-Ethylhexyl, 2-Ethylhexyl und 1-Propylpentyl;
- $C_1$-$C_4$-Halogenalkyl, $C_1$-$C_4$-Alkyl wie vorstehend genannt, das partiell oder vollständig durch Fluor, Chlor und/oder Brom substituiert ist, also z.B, Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluor-ethyl, 2-Chlor-2-fluorethyl, 2-Chlor-2,2-difluorethyl, 2,2-Dichlor-2-fluorethyl, 2,2,2-Trichlorethyl, Pentafluorethyl, 3-Chlorpropyl;
- $C_2$-$C_6$-Alkenyl: Ethenyl, Prop-1-en-1-yl, Prop-2-en-1-yl, 1-Methylethenyl, n-Buten-1-yl, n-Buten-2-yl, n-Buten-3-yl, 1-Methyl-prop-1-en-1-yl, 2-Methyl-prop-1-en-1-yl, 1-Methyl-prop-2-en-1-yl, 2-Methyl-prop-2-en-1-yl, n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-l-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl;

- $C_2$-$C_8$-Alkenyl: $C_2$-$C_6$-Alkenyl wie vorstehend genannt, sowie z.B. 1-(1-Methylethyl)-2-butenyl, 1-Butyl-2-butenyl, 1-Methyl-2-pentenyl und 1,4-Dimethyl-2-pentenyl;
- $C_3$-$C_6$-Alkinyl: Prop-1-in-1-yl, Prop-2-in-3-yl, n-But-1-in-1-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-1-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl;
- $C_2$-$C_8$-Alkinyl: insbesondere Ethinyl und $C_3$-$C_6$-Alkinyl wie vorstehend genannt;
- $C_3$-$C_6$-Cycloalkyl: Cyclopropyl, Cyclobutyl, Cyclopentyl und Cyclohexyl, vorzugsweise Cyclopropyl, Cylopentyl und Cyclohexyl;
- $C_5$-$C_8$-Bicycloalkyl: z.B. Bicyclo[4.1.0]hept-1-yl, Bicyclo[3.1.0]hex-1-yl, 2-($C_1$-$C_3$-Alkyl)-bicyclo[2.2.1]-hept-2-yl, 2-($C_1$-$C_3$-Alkyl)-bicyclo[2.2.2]oct-2-yl, wobei $C_1$-$C_3$-Alkyl jeweils für Methyl, Ethyl, n-Propyl und Isopropyl steht;
- $C_5$-$C_8$-Bicycloalkenyl: z.B. 2-($C_1$-$C_3$-Alkyl)-bicyclo[2.2.1]hept5-en-2-yl, 2-($C_1$-$C_3$-Alkyl)-bicyclo[2.2.2]-oct-5-en-2-yl, wobei $C_1$-$C_3$-Alkyl die vorstehend genannte Bedeutung hat;
- $C_3$-$C_6$-Cycloalkyl-$C_1$-$C_3$-alkyl: z.B. Cyclopropylmethyl, Cyclobutylmethyl, Cyclopentylmethyl, Cyclohexylmethyl, 2-(Cyclopropyl)ethyl, 2-(Cyclobutyl)ethyl, 2-(Cyclopentyl)ethyl, 2-(Cyclohexyl)ethyl, 3-(Cyclopropyl)propyl, 3-(Cyclobutyl)propyl, 3-(Cyclopentyl)propyl, 3-(Cyclohexyl)propyl;
- ($C_1$-$C_4$-Alkyl)carbonyl: Methylcarbonyl, Ethylcarbonyl, n-Propylcarbonyl, 1-Methyl-ethylcarbonyl, n-Butylcarbonyl, 1-Methylpropylcarbonyl, 2-Methylpropylcarbonyl und 1,1-Dimethylethylcarbonyl;
- $C_1$-$C_4$-Alkoxy: Methoxy, Ethoxy, n-Propoxy, 1-Methylethoxy, n-Butoxy, 1-Methyl-propoxy, 2-Methylpropoxy und 1,1-Dimethylethoxy;
- $C_1$-$C_4$-Alkylthio: Methylthio, Ethylthio, n-Propylthio, 1-Methylethylthio, n-Butylthio, 1-Methyl-propylthio, 2-Methylpropylthio und 1,1-Dimethylethylthio;
- ($C_2$-$C_4$-Alkoxy)carbonyl: Ethoxycarbonyl, n-Propoxycarbonyl, 1-Methylethoxycarbonyl, n-Butoxycarbonyl, 1-Methylpropoxycarbonyl, 2-Methylpropoxycarbonyl und 1,1-Dimethylethoxycarbonyl;
- ($C_1$-$C_4$-Alkoxy)carbonyl: Methoxycarbonyl und ($C_2$-$C_4$-Alkoxy)carbonyl wie vorstehend genannt;
- $C_1$-$C_4$-Alkoxy-$C_2$-$C_4$-alkyl: z.B. 2-Methoxyethyl, 2-Ethoxyethyl, 2-(n-Propoxy)ethyl, 2-(1-Methylethoxy)-ethyl, 2-(n-Butoxy)ethyl, 2-(1-Methylpropoxy)ethyl, 2-(2-Methylpropoxy)ethyl, 2-(1,1-Dimethylethoxy)-ethyl, 2-(Methoxy)propyl, 3-(Methoxy)propyl, 2-(Ethoxy)propyl, 3-(Ethoxy)propyl, 3-Propoxypropyl, 3-Butoxypropyl, 4-(Methoxy)butyl, 4-(Ethoxy)butyl, 4-(n-Propoxy)butyl und 4-(n-Butoxy)butyl;
- $C_3$-$C_4$-Alkenyloxy-$C_2$-$C_4$-alkyl: z.B. 2-(2-Ethenyl)ethyl, 2-(Prop-1-en-1-yl)ethyl, 2-(Prop-2-en-1-yl)ethyl, 2-(1-Methylethenyl)ethyl, 3-(2-Ethenyl)propyl, 3-(Prop-1-en-1-yl)propyl, 3-(Prop-2-en-1-yl)propyl, 3-(1-Methylethenyl)propyl, 4-(2-Ethenyl)butyl, 4-(Prop-1-en-1-yl)butyl, 4-(Prop-2-en-1-yl)butyl, 4-(1-Methylethenyl)butyl.

Im Hinblick auf die Verwendung der p-Phenylendiamin-Derivate I als Fungizide stehen

$R^1$ vorzugsweise für
- $C_3$-$C_8$-Alkyl, das gewünschtenfalls partiell oder vollständig halogeniert sein kann;
- $C_2$-$C_8$-Alkenyl, das gewünschtenfalls partiell oder vollständig halogeniert sein kann. Unter den halogenierten Alkenylresten ist 3-Chlor-2-propenyl besonders bevorzugt;
- Ethinyl, 2-Propinyl, 2-Butinyl oder 3-Butinyl, wobei diese vier Substituenten gewünschtenfalls partiell oder vollständig halogeniert sein können. Unter den halogenierten Alkinylresten sind 3-Chlor-2-propinyl, 3-Chlor-2-butinyl und 4-Chlor-3-butinyl ganz besonders bevorzugt;
- $C_3$-$C_6$-Cycloalkyl, das gewünschtenfalls ein oder zwei $C_1$-$C_4$-Alkylreste tragen kann;
- Bicyclo[4.1.0]hept-1-yl, Bicyclo[3.1.0]hex-1-yl, 2-($C_1$-$C_3$-Alkyl)-bicyclo[2.2.2]oct-2-yl, 2-($C_1$-$C_3$-Alkyl)-bicyclo[2.2.2]oct-5-en-2-yl, 2-($C_1$-$C_3$-Alkyl)-bicyclo[2.2.1]hept-2-yl, 2-($C_1$-$C_3$-Alkyl)-bicyclo[2.2.1]hept-5-en-2-yl, 6-($C_1$-$C_3$-alkyl)-5,6-dihydro-4H-pyran-6-yl oder 2-($C_1$-$C_3$-Alkyl)-2.3.5.6-tetrahydro-4H-pyran-2-yl;

$R^2$ vorzugsweise für Wasserstoff oder, gemeinsam mit $R^1$ und der CO-N-Gruppe, einen Azetidin-2-on-Ring bildet, der eine Methylgruppe tragen kann. Ganz besonders bevorzugt ist Wasserstoff;

$R^3$ vorzugsweise für
- $C_1$-$C_6$-Alkyl, $C_2$-$C_6$-Alkenyl, $C_3$-$C_6$-Alkinyl, $C_3$-$C_6$-Cycloalkyl;
- Cyclopropylmethyl oder Cyclobutylmethyl;

$R^4$ vorzugsweise für Wasserstoff, $C_1$-$C_6$-Alkyl, $C_2$-$C_6$-Alkenyl, $C_3$-$C_6$-Alkinyl oder $C_3$-$C_6$-Cyclo-

alkyl;

| | |
|---|---|
| $R^a$ und $R^b$ | vorzugsweise für Wasserstoff; |
| $R^c$ | vorzugsweise für<br>- Cyano, Nitro, Halogen;<br>- $C_1$-$C_4$-Alkyl, insbesondere Methyl oder Ethyl;<br>- Trifluormethyl, Methoxycarbonyl, Acetyl oder Formyl; |
| $R^d$ | vorzugsweise für Wasserstoff oder Halogen, insbesondere für Wasserstoff oder Fluor; |
| X und Y | vorzugsweise für Sauerstoff. |

In der folgenden Tabelle 1 sind ganz besonders bevorzugte p-Phenylendiamin-Derivate I aufgeführt

Tabelle 1

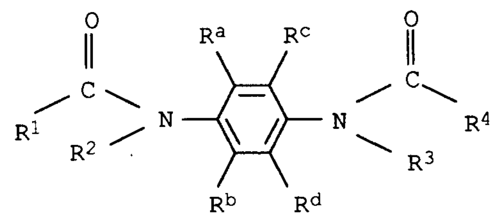

I (X, Y = Sauerstoff)

| Nr. | $R^1$ | $R^2$ | $R^a$ | $R^c$ | $R^b$ | $R^d$ | $R^3$ | $R^4$ |
|---|---|---|---|---|---|---|---|---|
| 01 | $CH(CH_3)_2$ | H | H | Cl | H | H | $CH_3$ | H |
| 02 | $CH(CH_3)_2$ | H | H | Cl | H | H | $CH_3$ | $CH_3$ |
| 03 | $CH(CH_3)_2$ | H | H | Cl | H | H | $CH_3$ | $C_2H_5$ |
| 04 | $C(CH_3)_3$ | H | H | Cl | H | H | $CH_3$ | $CH_3$ |
| 05 | $C(CH_3)_3$ | H | H | $CH_3$ | H | H | $CH_3$ | $CH_3$ |
| 06 | $C(CH_3)_3$ | H | H | Cl | H | F | $CH_3$ | $CH_3$ |
| 07 | $C(CH_3)_3$ | H | F | F | H | F | $CH_3$ | $CH_3$ |
| 08 | $C(CH_3)_3$ | H | H | F | H | F | $CH_3$ | $C_2H_5$ |
| 09 | $C(CH_3)_3$ | H | H | Br | H | F | $CH_3$ | $CH_3$ |
| 10 | $C(CH_3)_3$ | H | H | J | H | H | $CH_3$ | $CH_3$ |
| 11 | $C(CH_3)_3$ | H | H | H | H | Cl | $C_2H_5$ | $CH_3$ |
| 12 | $C(CH_3)_3$ | H | H | H | H | Cl | $CH_2-CH=CH_2$ | $CH_3$ |

| Nr. | $R^1$ | $R^2$ | $R^a$ | $R^c$ | $R^b$ | $R^d$ | $R^3$ | $R^4$ |
|---|---|---|---|---|---|---|---|---|
| 13 | $C(CH_3)_3$ | H | H | H | H | Cl | Cyclopropyl | $CH_3$ |
| 14 | Cyclopropyl | H | H | H | H | Cl | $CH_3$ | $C_2H_5$ |
| 15 | 1-$CH_3$-cycloprop-1-yl | H | H | H | H | Cl | $CH_3$ | $CH_3$ |
| 16 | 1-$CH_3$-cycloprop-1-yl | H | H | H | H | Cl | $C_2H_5$ | $CH_3$ |
| 17 | 1-$CH_3$-cyclopent-1-yl | H | H | Cl | H | H | $CH_3$ | $CH_3$ |
| 18 | 1-$CH_3$-cyclophex-1-yl | H | H | Cl | H | H | $CH_3$ | $CH_3$ |
| 19 | 1-$CH_3$-cyclohex-1-yl | H | H | H | H | Cl | $CH_3$ | $CF_3$ |
| 20 | 1-$CH_3$-cyclohex-1-yl | H | H | H | H | $CH_3$ | $CH_3$ | $CH_3$ |
| 21 | 1-$CH_3$-cyclohex-1-yl | H | H | H | H | Cl | $CH_3$ | $C_2H_5$ |
| 22 | 1-$CH_3$-cyclohex-1-yl | H | H | Cl | H | H | Cyclopropyl | $CH_3$ |
| 23 | $C(CH_3)_2-C_2H_5$ | H | H | Cl | H | H | $CH_3$ | $CH_3$ |
| 24 | $C(CH_3)_2-C_3H_7$ | H | H | H | H | Cl | $CH_3$ | $C_2H_5$ |
| 25 | $CF(CH_3)_2$ | H | H | Cl | H | H | $CH_3$ | $CH_3$ |
| 26 | $CCl(CH_3)_2$ | H | H | Cl | H | H | $CH_3$ | $CH_3$ |
| 27 | $C(CH_3)_2-CH_2Cl$ | H | H | H | H | Cl | $CH_3$ | $CH_3$ |
| 28 | $C(CH_3)_3$ | H | H | H | H | $CF_3$ | $CH_3$ | $CH_3$ |
| 29 | $C(CH_3)_3$ | H | H | H | H | $CH=CH_2$ | $C_2H_5$ | $CH_3$ |
| 30 | $C(CH_3)_3$ | H | H | H | H | $C\equiv CH$ | $CH_3$ | $CH_3$ |
| 31 | $C(CH_3)_3$ | H | H | $NO_2$ | H | H | $C_2H_5$ | $CH_3$ |
| 32 | $C(CH_3)_3$ | H | H | CN | H | H | $CH_3$ | $C_2H_5$ |
| 33 | $C(CH_3)_3$ | H | H | H | H | $COCH_3$ | $CH_3$ | $CH_3$ |
| 34 | $C(CH_3)_3$ | H | H | $OCH_3$ | H | H | $C_2H_5$ | $CH_3$ |

| Nr. | $R^1$ | $R^2$ | $R^a$ | $R^c$ | $R^b$ | $R^d$ | $R^3$ | $R^4$ |
|---|---|---|---|---|---|---|---|---|
| 35 | $C(CH_3)_3$ | H | H | H | H | CHO | $C_2H_5$ | $CH_3$ |
| 36 | $C(CH_3)_3$ | H | H | Cl | H | F | $C_2H_5$ | $CH_3$ |
| 37 | $C(CH_3)_3$ | H | H | Cl | H | F | $CH_3$ | $CH_3$ |
| 38 | $C(CH_3)_3$ | H | H | Cl | H | H | $CH_2-CH=CH_2$ | $CH_3$ |
| 39 | $C(CH_3)_3$ | H | H | Cl | H | H | $CH_2-C\equiv CH$ | $CH_3$ |
| 40 | $C(CH_3)_3$ | H | H | H | H | Cl | Cyclopropyl | $CH_3$ |
| 41 | $C(CH_3)_3$ | H | H | Cl | H | H | Cyclopropyl | $CF_3$ |
| 42 | $C(CH_3)_3$ | H | H | Cl | H | H | $CH_3$ | $CCl_3$ |
| 43 | $C(CH_3)_3$ | H | H | H | H | Cl | $CH_3$ | $CH_2Cl$ |
| 44 | $C(CH_3)_3$ | H | H | Cl | H | H | $CH_2CF_3$ | $CH_3$ |
| 45 | $C(CH_3)_2-CH_2OCH_3$ | H | H | Cl | H | H | $C_2H_5$ | $CH_3$ |
| 46 | $C(CH_3)_2-CH_2F$ | H | H | H | H | Cl | $CH_3$ | $CH_3$ |
| 47 | $C(CH_3)_2-CH_2CN$ | H | H | H | Cl | H | $CH_3$ | $CH_3$ |
| 48 | Bicyclo[4.1.0]hept-1-yl | H | H | H | H | Cl | $CH_3$ | $CH_3$ |
| 49 | Bicyclo[4.1.0]hept-1-yl | H | H | Cl | H | H | $CH_3$ | $C_2H_5$ |
| 50 | Bicyclo[4.1.0]hept-1-yl | H | H | Cl | H | F | $CH_3$ | $CH_3$ |
| 51 | Bicyclo[4.1.0]hept-1-yl | H | H | $CH_3$ | H | H | $CH_3$ | $CH_3$ |
| 52 | Bicyclo[4.1.0]hept-1-yl | H | H | Cl | H | H | Cyclopropyl | $CH_3$ |
| 53 | Bicyclo[4.1.0]hept-1-yl | H | H | H | H | Cl | $CH_3$ | $CF_3$ |
| 54 | Bicyclo[4.1.0]hept-1-yl | H | H | Cl | H | H | $CH_2-CH=CH_2$ | $CH_3$ |
| 55 | Bicyclo[4.1.0]hept-1-yl | H | H | Cl | H | H | $CH_3$ | $CHCl_2$ |
| 56 | Bicyclo[3.1.0]hex-1-yl | H | H | H | H | Cl | $CH_3$ | $CH_3$ |

| Nr. | $R^1$ | $R^2$ | $R^a$ | $R^c$ | $R^b$ | $R^d$ | $R^3$ | $R^4$ |
|---|---|---|---|---|---|---|---|---|
| 57 | Bicyclo[3.1.0]hex-1-yl | H | H | H | H | Cl | $C_2H_5$ | $CH_3$ |
| 58 | Bicyclo[3.1.0]hex-1-yl | H | H | $CH_3$ | H | H | $C_2H_5$ | $CH_3$ |
| 59 | 2-$CH_3$-bicyclo[2.2.2]oct-2-yl | H | H | H | H | Cl | $CH_3$ | $CH_3$ |
| 60 | 2-$CH_3$-bicyclo[2.2.2]oct-2-yl | H | H | $CH_3$ | H | H | $CH_3$ | $CH_3$ |
| 61 | 2-$CH_3$-bicyclo[2.2.2]oct-2-yl | H | H | Cl | H | H | $CH_3$ | $CF_3$ |
| 62 | 2-$CH_3$-bicyclo[2.2.2]oct-2-yl | H | H | Cl | H | H | $CH_3$ | $C_2H_5$ |
| 63 | 2-$CH_3$-bicyclo[2.2.2]oct-2-yl | H | H | H | H | Cl | $C_2H_5$ | $CH_3$ |
| 64 | 2-$CH_3$-bicyclo[2.2.2]oct-2-yl | H | H | H | H | H | $CH_2$-CH=$CH_2$ | $CH_3$ |
| 65 | 2-$CH_3$-bicyclo[2.2.2]oct-5-en-2-yl | H | H | Cl | H | H | $CH_3$ | $CH_3$ |
| 66 | 2-$CH_3$-bicyclo[2.2.2]oct-5-en-2-yl | H | H | Cl | H | H | $C_2H_5$ | $CH_3$ |
| 67 | 2-$CH_3$-bicyclo[2.2.2]oct-5-en-2-yl | H | H | H | H | $CH_3$ | $CH_3$ | $CH_3$ |
| 68 | 2-$CH_3$-bicyclo[2.2.1]hept-2-yl | H | H | H | H | Cl | $CH_3$ | $CH_3$ |
| 69 | 2-$CH_3$-bicyclo[2.2.1]hept-2-yl | H | H | H | H | Cl | $C_2H_5$ | $CH_3$ |
| 70 | 2-$CH_3$-bicyclo[2.2.1]hept-2-yl | H | H | Cl | H | H | $CH_3$ | $CF_3$ |
| 71 | 2-$CH_3$-bicyclo[2.2.1]hept-2-yl | H | H | Cl | H | H | $CH_2$-CH=$CH_2$ | $CH_3$ |
| 72 | 2-$CH_3$-bicyclo[2.2.1]hept-2-yl | H | H | Cl | H | H | Cyclopropyl | $CH_3$ |
| 73 | 2-$CH_3$-bicyclo[2.2.1]hept-2-yl | H | H | H | H | Cl | $CH_3$ | $C_2H_5$ |
| 74 | 2-$CH_3$-bicyclo[2.2.1]hept-2-yl | H | H | H | H | Cl | i-$C_3H_7$ | $CH_3$ |
| 75 | 2-$CH_3$-bicyclo[2.2.1]hept-2-yl | H | H | Cl | H | H | $CH_3$ | $CHCl_2$ |
| 76 | 2-$CH_3$-bicyclo[2.2.1]hept-2-yl | H | H | $CH_3$ | H | H | $CH_3$ | $CH_3$ |
| 77 | 2-$CH_3$-bicyclo[2.2.1]hept-2-yl | H | H | Cl | H | F | $CH_3$ | $CH_3$ |
| 78 | 2-$CH_3$-bicyclo[2.2.1]hept-2-yl | H | H | Cl | H | H | $CH_3$ | Cyclopropyl |

| Nr. | $R^1$ | $R^2$ | $R^a$ | $R^c$ | $R^b$ | $R^d$ | $R^3$ | $R^4$ |
|---|---|---|---|---|---|---|---|---|
| 79 | 2-$CH_3$-bicyclo[2.2.1]hept-2-yl | H | H | H | H | Cl | $CH_2-C\equiv CH$ | $CH_3$ |
| 80 | 2-$CH_3$-bicyclo[2.2.1]hept-5-en-2-yl | H | H | Cl | H | H | $CH_3$ | $CH_3$ |
| 81 | 2-$CH_3$-bicyclo[2.2.1]hept-5-en-2-yl | H | H | Cl | H | H | $C_2H_5$ | $CH_3$ |
| 82 | 2-$CH_3$-bicyclo[2.2.1]hept-5-en-2-yl | H | H | Cl | H | H | Cyclopropyl | $CH_3$ |
| 83 | 2-$CH_3$-bicyclo[2.2.1]hept-5-en-2-yl | H | H | H | H | Cl | $CH_3$ | $CF_3$ |
| 84 | 2-$CH_3$-bicyclo[2.2.1]hept-5-en-2-yl | H | H | H | H | Cl | $CH_2-CH=CH_2$ | $CH_3$ |
| 85 | 2-$CH_3$-bicyclo[2.2.1]hept-5-en-2-yl | H | H | Cl | H | F | $CH_3$ | $CH_3$ |
| 86 | 2-$CH_3$-bicyclo[2.2.1]hept-5-en-2-yl | H | H | Cl | H | H | i-$C_3H_5$ | $CH_3$ |
| 87 | 2-$CH_3$-bicyclo[2.2.1]hept-5-en-2-yl | H | H | H | H | Cl | $CH_3$ | Cyclopropyl |
| 88 | 6-$CH_3$-5,6-dihydro-4-H-pyran-6-yl | H | H | Cl | H | H | $CH_3$ | $CH_3$ |
| 89 | 6-$CH_3$-5,6-dihydro-4-H-pyran-6-yl | H | H | Cl | H | H | $C_2H_5$ | $CH_3$ |
| 90 | 6-$CH_3$-5,6-dihydro-4-H-pyran-6-yl | H | H | H | H | Cl | Cyclopropyl | $CH_3$ |
| 91 | 6-$CH_3$-5,6-dihydro-4-H-pyran-6-yl | H | H | H | H | Cl | i-$C_3H_7$ | $CH_3$ |
| 92 | 6-$CH_3$-5,6-dihydro-4-H-pyran-6-yl | H | H | Cl | H | H | $CH_3$ | $CF_3$ |
| 93 | 2-$CH_3$-2.3.5.6-tetrahydro-4H-pyran-2-yl | H | H | H | H | Cl | $CH_3$ | $CH_3$ |
| 94 | 2-$CH_3$-2.3.5.6-tetrahydro-4H-pyran-2-yl | H | H | Cl | H | F | $CH_3$ | $CH_3$ |
| 95 | 2-$CH_3$-2.3.5.6-tetrahydro-4H-pyran-2-yl | H | H | H | H | $CH_3$ | $CH_3$ | $CH_3$ |
| 96 | 2-$CH_3$-2.3.5.6-tetrahydro-4H-pyran-2-yl | H | H | H | H | Cl | Cyclopropyl | $CH_3$ |
| 97 | 2-$CH_3$-2.3.5.6-tetrahydro-4H-pyran-2-yl | H | H | H | H | Cl | $CH_3$ | $CF_3$ |
| 98 | 2-$CH_3$-2.3.5.6-tetrahydro-4H-pyran-2-yl | H | H | Cl | H | H | $CH_3$ | $CHCl_2$ |
| 99 | 2-$CH_3$-2.3.5.6-tetrahydro-4H-pyran-2-yl | H | H | Cl | H | H | $CH_3$ | $CCl_3$ |
| 100 | 2-$CH_3$-2.3.5.6-tetrahydro-4H-pyran-2-yl | H | H | H | H | Cl | $CH_3$ | Cyclopropyl |

| Nr. | $R^1$ | $R^2$ | $R^a$ | $R^c$ | $R^b$ | $R^d$ | $R^3$ | $R^4$ |
|---|---|---|---|---|---|---|---|---|
| 101 | 2-$CH_3$-2.3.5.6-tetrahydro-4H-pyran-2-yl | H | H | H | H | Cl | $CH_2$-CH=$CH_2$ | $CH_3$ |
| 102 | 2-$CH_3$-2.3.5.6-tetrahydro-4H-pyran-2-yl | H | H | Cl | H | H | $CH_3$ | $C_2H_5$ |
| 103 | $C(CH_3)_3$ | H | H | H | H | CN | $CH_3$ | $CH_3$ |
| 104 | 1-$CH_3$-cycloprop-1-yl | H | H | H | H | CN | $CH_3$ | $CH_3$ |
| 105 | 1-$CH_3$-cyclohex-1-yl | H | H | H | H | CN | $CH_3$ | $CH_3$ |
| 106 | Bicyclo[4.1.0]hept-1-yl | H | H | H | H | CN | $CH_3$ | $CH_3$ |
| 107 | $C(CH_3)_3$ | H | H | $CF_3$ | H | H | $CH_3$ | $CF_3$ |
| 108 | 1-$CH_3$-cycloprop-1-yl | H | H | $CF_3$ | H | H | $CH_3$ | $CH_3$ |
| 109 | 1-$CH_3$-cyclohex-1-yl | H | H | $CF_3$ | H | H | $CH_3$ | $CH_3$ |
| 110 | Bicyclo[4.-1.0]hept-1-yl | H | H | $CF_3$ | H | H | $CH_3$ | $CH_3$ |
| 111 | $C(CH_3)_3$ | H | H | Cl | H | Cl | $CH_3$ | $CH_3$ |
| 112 | 1-$CH_3$-cycloprop-1-yl | H | H | Cl | H | Cl | $CH_3$ | $CH_3$ |
| 113 | 1-$CH_3$-cyclohex-1-yl | H | H | Cl | H | Cl | $CH_3$ | $CH_3$ |
| 114 | Bicyclo[4.1.0]hept-1-yl | H | H | Cl | H | Cl | $CH_3$ | $CH_3$ |
| 115 | $C(CH_3)_3$ | H | H | H | H | Cl | $CH(CH_3)_2$ | $CH_3$ |
| 116 | $C(CH_3)_3$ | H | H | H | H | Cl | $CH_3$ | $CHCl_2$ |
| 117 | $C(CH_3)_3$ | H | H | H | H | Cl | $CH_3$ | $CH_2Cl$ |
| 118 | $C(CH_3)_3$ | H | H | H | H | Cl | $CH_3$ | $CH(CH_3)$-Cl |

Die p-Phenylendiamin-Derivate I sind nach verschiedenen Methoden erhältlich, vorzugsweise auf an sich bekannte Weise durch Acylierung von p-Amino-acetaniliden III mit Carbonsäurehalogeniden $R^1$-CX-$Hal^1$ in Gegenwart von Base (vgl. z.B. J. March, Advanced Organic Chemistry, 2nd Ed., 382f., McGraw-Hill, 1977):

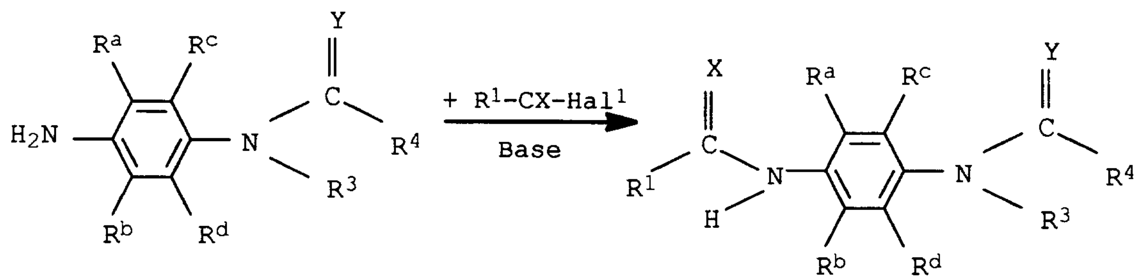


III

I ($R^2$=H)

$Hal^1$ = Halogen wie Fluor, Chlor, Brom und Jod, insbesondere Chlor.

Die Umsetzung erfolgt üblicherweise bei Temperaturen von (-20) bis 100 °C, vorzugsweise 0 bis 50 °C.

Geeignete Lösungsmittel sind beispielsweise aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid. Besonders bevorzugt sind Toluol, Xylol und Methylenchlorid.

Es können auch Gemische der genannten Lösungsmittel verwendet werden.

Als Basen kommen allgemein anorganische Verbindungen, z.B. Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calziumoxid und Magnesiumoxid, Alkalimetall- und Erdalkalimetallcarbonate wie Lithiumcarbonat und Calziumcarbonat, oder Alkalimetallhydrogencarbonate wie Natriumhydrogencarbonat, und organische Basen, z.B. tertiäre Amine wie Trimethylamin, Triethylamin, Triisopropylethylamin und N-Methylpiperidin, Pyridin und substituierte Pyridine wie Collidin, Lutidin und 4-Dimethylaminopyridin, oder bicyclische Amine, in Betracht.

Besonders bevorzugt sind Triethylamin und Pyridin.

Die Base wird im allgemeinen in äquimolaren Mengen eingesetzt, bezogen auf die Menge an VII. Sie kann aber auch in einem Überschuß von z.B. 5 bis 30 mol-%, vorzugsweise 5 bis 10 mol-%, eingesetzt werden.

Bei Verwendung von teriären Aminen als Basen besteht die Möglichkeit, ohne Lösungsmittel in einem noch größeren Überschuß an Amin zu arbeiten.

Die Edukte werden normalerweise in äquimolaren Mengen miteinander umgesetzt. Im Hinblick auf die Ausbeute kann es jedoch vorteilhaft sein, III in einem Überschuß von etwa 1 bis 20 mol-%, vorzugsweise 1 bis 10 mol-%, bezogen auf VII einzusetzen.

Durch Acylierung von III mit $\beta$-Halogenpropionsäurehalogeniden, die in $\alpha$-Position einen oder zwei $C_1$-$C_4$-Alkylreste tragen, lassen sich p-Phenylendiamin-Derivaten I mit X = Sauerstoff und $R^1$ = 2-[1-(Halogen)-$C_3$-$C_6$-alkyl] oder 2-[1-(Halogen)-2-($C_1$-$C_4$-alkyl)-$C_3$-$C_6$-alkyl] herstellen. Aus diesen Verbindungen kann mittels anorganischer Basen wie Natriumhydroxid Halogenwasserstoff abgespalten werden (vgl. hierzu z.B. J. Chem. Soc. Chem. Commun., 903 (1978)), wobei man p-Phenylendiamin-Derivate I erhält, bei denen $R^2$ gemeinsam mit dem $R^1$-CX-N- Teil einen Azetidin-2-on Ring bedeutet, der ein oder zwei $C_1$-$C_4$-Alkylreste trägt.

Die Cycliserung zum Azetidin-2-on Ring erfolgt besonders vorteilhaft unter den Bedingungen der Phasentransfer-Katalyse (vgl. z.B. Synthesis 1976, 113), wobei das Produkt der vorangehenden Acylierung in organischer Phase, z.B. in Methylenchlorid, und die Base in wässriger Lösung, z.B. in Form von Natronlauge, eingesetzt wird.

Die Carbonsäurehalogenide $R^1$-CX-Hal oder die entsprechenden Carbonsäuren sind allgemein bekannt. Bicyclische Carbonsäuren sind nach an sich bekannten Methoden herstellbar (vgl. z.B. J. Org. Chem. 35, 2666 (1970); J. Org. Chem. 50, 2128 (1985), Houben-Weyl, Methoden der Org. Chemie, Bd. 6/4, S. 78 und dto., Bd. 5/1c, S. 991 ff sowie U.S. 2,942,026).

Die p-Amino-acetanilide der Formel III

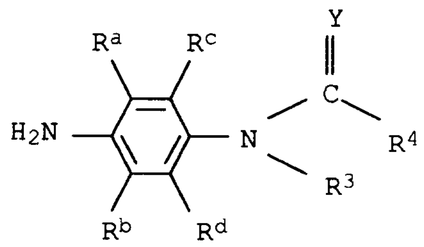

III

in der die Substituenten folgende Bedeutung haben:

- $R^3$ $C_1$-$C_6$-Alkyl, $C_2$-$C_6$-Alkenyl, $C_3$-$C_6$-Alkinyl, $C_3$-$C_6$-Cycloalkyl, $C_3$-$C_6$-Cycloalkyl-$C_1$-$C_3$-alkyl, $C_1$-$C_4$-Alkoxy-$C_2$-$C_4$-alkyl, $C_3$-$C_4$-Alkenyloxy-$C_2$-$C_4$-alkyl;
- $R^4$ Wasserstoff, $C_1$-$C_6$-Alkyl, $C_2$-$C_6$-Alkenyl, $C_3$-$C_6$-Alkinyl, $C_3$-$C_6$-Cycloalkyl, durch $C_1$-$C_3$-Alkyl substituiertes $C_3$-$C_6$-Cycloalkyl, wobei diese Gruppen jeweils 1 - 3 Halogenatome tragen können.
- $R^a$, $R^b$ Wasserstoff oder Fluor;
- $R^c$, $R^d$ Wasserstoff, Cyano, Nitro Halogen, $C_1$-$C_4$-Alkyl, $C_1$-$C_4$-Alkoxy, $C_1$-$C_4$-Halogenalkyl, ($C_1$-$C_4$-Alkoxy)carbonyl, $C_1$-$C_4$-Alkylcarbonyl, oder Formyl,mit der Maßgabe, daß $R^c$ und $R^d$ nicht gleichzeitig Wasserstoff sein können;
- Y Sauerstoff oder Schwefel

sind neu. Man erhält sie z.B. durch Reduktion von 4-Nitroacetaniliden der Formel VI in an sich bekannter Weise mit z.B. Eisen in Eisessig, in Eisessig-Alkohol- oder Alkohol-Wasser-Gemischen als Reduktionsmittel oder durch katalytische Reduktion mit Wasserstoff an z.B. Edelmetallkatalysatoren wie Palladium und Platin.

Die 4-Nitroacetanilide VI sind ihrerseits erhältlich, indem man z.B. Nitrohalogenverbindungen der Formel IV auf an sich bekannte Weise mit primären Aminen der Formel $NH_2$-$R^3$ umsetzt (vgl. z.B. Rec. trav. Chim. Pays-Bas 51, 878 (1932) und EP-A 430 847) und die erhaltenen Nitroaniline der Formel V anschließend auf an sich bekannte Weise acyliert, gegebenenfalls in Gegenwart von Base (vgl. z.B. J. March, Advanced Organic Chemistry, 2nd Ed., 382f., McGraw-Hill, 1977).

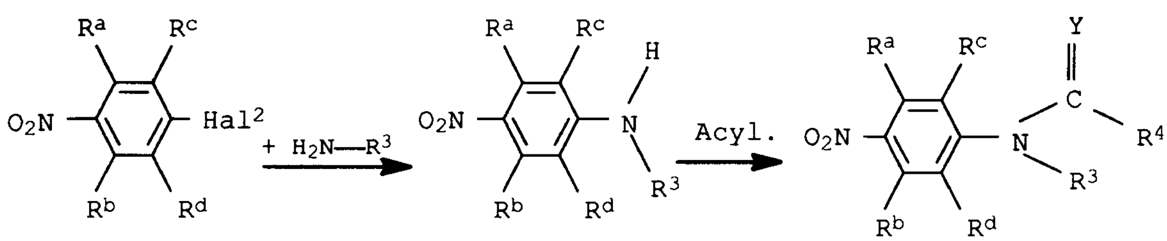


IV V VI

$Hal^2$ = Halogen wie Fluor, Chlor, Brom und Jod, insbesondere Fluor oder Chlor.

Als Acylierungsmittel kommen z.B. Anhydride der Formel $(R^4CO)_2O$oder Säurechloride der Formel $R^4$-CY-Cl in Betracht.

Als Basen sind organische Basen wie tertiäre Amine und Pyridinderivate besonders vorteilhaft.

Eine Variante der Acylierung besteht darin, die Nitroaniline V erst mittels Alkalimetall- oder Erdalkalimetallalkoholaten, vorzugsweise Kalium-tert.-butanolat, oder mittels Alkalimetallalkylen, vorzugsweise Butyllithium, in die entsprechenden Salze zu überführen und diese dann zu acylieren.

Für die Salzbildung haben sich dipolare aprotische Lösungsmittel wie Dimethylformamid als gut geeignet erwiesen.

Die Acylierung wird vorteilhaft bei einer Temperatur von (-50) bis 0°C vorgenommen, wobei die Anhydride $(R^4CO)_2O$ als Acylierungsmittel besonders bevorzugt sind.

Die Herstellung derjenigen 4-Nitroacetanilide VI, bei denen Y Sauerstoff bedeutet, kann auch aus Nitroanilinen der Formel VII erfolgen, indem diese auf eine der vorstehend genannten Weisen mit Anhydriden der Formel $(R^4CO)_2O$ oder Säurechloriden $R^4COCl$ in Gegenwart einer organischen Base acyliert werden und man das jeweilige Reaktionsprodukt VIII mit $R^3$-Hal oder $R^3O$-$SO_2$-$OR^3$ alkyliert:

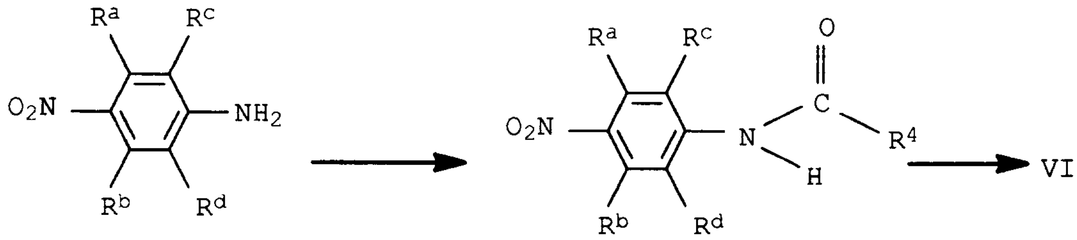

VII VIII (Y=O)

Die Alkylierung erfolgt normalerweise in Gegenwart einer starken Base, z.B. eines Metallhydrides oder Metallalkyls wie Butyl-lithium in einem aprotischen Lösungsmittel wie Dimethylformamid, oder unter Bedingungen der Phasentransfer-Katalyse (vgl. hierzu z.B. Synthesis 1976, 113).

Die 4-Nitroacetanilide VI, bei denen Y Sauerstoff bedeutet, und die p-Phenylendiamin-Derivate I, bei denen X und Y Sauerstoff bedeuten, können mit üblichen Schwefelungsreagenzien wie Phosphor(V)-sulfid ($P_4S_{10}$) und 2,4-Bis(4-methoxyphenyl)-1,3,2,4-dithiadiphosphetan-2,4-dithion ("Lawessons Reagenz") in 4-Nitroacetanilide VI mit Y = Schwefel überführt werden.

Die Umsetzung erfolgt in der Regel in einem inerten Lösungsmittel, beispielsweise in einem aromatischen Kohlenwasserstoff wie Toluol und o-, m-, p-Xylol, in einem Ether wie Diethylether, 1,2-Dimethoxyethan und Tetrahydrofuran, oder in einem organischen Amin wie Pyridin.

Die Menge an Schwefelungsreagenz ist nicht kritisch; normalerweise verwendet man die 1- bis 5-fache molare Menge, bezogen auf die zu schwefelnde Verbindung VI oder I.

Normalerweise liegt die Reaktionstemperatur zwischen 20 und 200 °C, vorzugsweise zwischen 40 °C und dem Siedepunkt des Lösungsmittels.

Alle vorstehend beschriebenen Reaktionen werden zweckmäßigerweise bei Atmosphärendruck oder unter dem Eigendruck des jeweiligen Verdünnungsmittels vorgenommen.

Die p-Phenylendiamin-Derivate der Formel I eignen sich als Fungizide.

Herstellungsbeispiel

2-Chlor-4-pivaloyl-amido-N-methylacetanilid (Verbindung Nr. I.02, Tabelle 2)

Zu einer Lösung von 1,98 g (0,01 mol) 2-Chlor-4-amino-N-methylacetanilid und 1,21 g (0,012 mol) Triethylamin in 20 ml Dichlormethan tropfte man bei 0 °C 1,45 g (0,012 mol) Pivaloylchlorid. Die Reaktionsmischung wurde anschließend 1 Std. bei 0 °C und 12 Stunden bei ca. 20 °C gerührt, danach zweimal mit je 30 ml Wasser gewaschen und schließlich getrocknet. Nach Verdampfen des Lösungsmittels wurde der Rückstand mit Methyl-tert.-butylether ausgekocht. Ausbeute: 2,10 g (73 %); Fp.: 160-162 °C.

Vorstufe a) 2-Chlor-4-nitro-N-methylacetanilid

10,0 g (0,054 mol) 2-Chlor-4-nitro-N-mehylanilin (Herstellung siehe Rec. trav. Chim. P.B. 51, 878 (1932)) in 100 ml Acetanhydrid wurden 2 Std. auf Rükflußtemperatur erhitzt, wonach man das Acetanhydrid bei reduziertem Druck verdampfte. Der Rückstand, gelöst in 60 ml Methyl-tert.-butylether, wurde mit 40 ml Wasser versetzt. Anschließend neutralisierte man die wässrige Phase mit festem Natriumhydrogencarbonat. Die organische Phase wurde abgetrennt, getrocknet und eingeengt. Ausbeute: 10,8 g (88 %); Fp.: 84-86 °C.

Vorstufe b) 2-Chlor-4-amino-N-methylacetanilid

Zu einer Suspension von 7,9 g (0,14 mol) Eisenpulver in 40 ml Eisessig tropfte man unter Rühren bei 70 °C eine warme Lösung von 10,8 g (0,047 mol) 2-Chlor-4-nitro-N-methylacetanilid in 80 ml Methanol, wonach die Mischung 3 Std. auf Rückflußtemperatur erhitzt wurde. Nach dem Abkühlen gab man 80 ml Essigester und 320 ml Eiswasser zu. Man rührte noch 30 Minuten nach und trennte dann den Feststoffanteil und die Essigester-Phase ab. Der Feststoff wurde noch 2 mal mit je 100 ml Essigester gewaschen. Die vereinigten Essigester-Phasen wurden mit 50 ml gesättigter Natriumhydrogencarbonat-Lösung gegengewaschen, dann getrocknet und eingeengt. Ausbeute: 8,5 g (90 %) als Öl.

In Tabelle 2 sind weitere p-Phenylendiamin-Derivate I enthalten, die sich analog zu der Beispielsverbindung herstellen lassen:

Tabelle 2

Verbindungen der Formel I

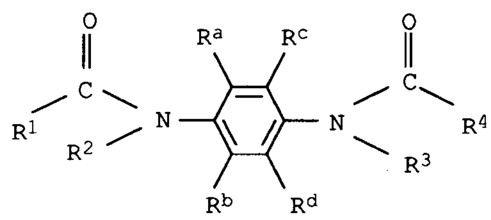

I (X,Y = Sauerstoff)

| Nr. | $R^1$ | $R^2$ | $R^a$ | $R^c$ | $R^b$ | $R^d$ | $R^3$ | $R^4$ | Fp. [°C] |
|---|---|---|---|---|---|---|---|---|---|
| I.01 | $CH(CH_3)_2$ | H | H | H | H | Cl | $CH_3$ | $CH_3$ | 156-158 |
| I.02 | $C(CH_3)_3$ | H | H | H | H | Cl | $CH_3$ | $CH_3$ | 160-162 |
| I.03 | $C(C_2H_5)(CH_3)_2$ | H | H | H | H | Cl | $CH_3$ | $CH_3$ | 159-163 |
| I.04 | $C(CH_3)_2-CH_2Cl$ | H | H | H | H | Cl | $CH_3$ | $CH_3$ | 169-171 |
| I.05 | $C(CH_3)_2-Cl$ | H | H | H | H | Cl | $CH_3$ | $CH_3$ | 133-135 |
| I.06 | Cyclopropyl | H | H | H | H | Cl | $CH_3$ | $CH_3$ | 181-185 |
| I.07 | Cyclobutyl | H | H | H | H | Cl | $CH_3$ | $CH_3$ | 150-155 |
| I.08 | Cyclopentyl | H | H | H | H | Cl | $CH_3$ | $CH_3$ | 186-189 |
| I.09 | Cyclohexyl | H | H | H | H | Cl | $CH_3$ | $CH_3$ | 150-151 |
| I.10 | 1-$CH_3$-cycloprop-1-yl | H | H | H | H | Cl | $CH_3$ | $CH_3$ | 147-149 |
| I.11 | 1-$CH_3$-cyclohex-1-yl | H | H | H | H | Cl | $CH_3$ | $CH_3$ | 175-178 |

| Nr. | $R^1$ | $R^2$ | $R^a$ | $R^c$ | $R^b$ | $R^d$ | $R^3$ | $R^4$ | Fp. [°C] |
|---|---|---|---|---|---|---|---|---|---|
| I.12 | Bicyclo[4.1.0]hept-1-yl | H | H | Cl | H | H | $CH_3$ | $CH_3$ | 150-152 |
| I.13 | 2-$CH_3$-bicyclo[2.2.1]-hept-2-yl | H | H | Cl | H | H | $CH_3$ | $CH_3$ | 221-222 |
| I.14 | 2-$CH_3$-2,3,5,6-tetra-pyran-4H-pyran-2-yl | H | H | Cl | H | H | $CH_3$ | $CH_3$ | |
| I.15 | $C(CH_3)_3$ | H | H | H | H | $CH_3$ | $CH_3$ | $CH_3$ | 145-147 |
| I.16 | 1-$CH_3$-cycloprop-1-yl | H | H | H | H | $CH_3$ | $CH_3$ | $CH_3$ | |
| I.17 | 1-$CH_3$-cyclohex-1-yl | H | H | H | H | $CH_3$ | $CH_3$ | $CH_3$ | 132-134 |
| I.18 | $C(CH_3)_3$ | H | H | H | H | $CF_3$ | $CH_3$ | $CH_3$ | 138-141 |
| I.19 | 1-$CH_3$-cycloprop-1-yl | H | H | H | H | $CF_3$ | $CH_3$ | $CH_3$ | 139-142 |
| I.20 | 1-$CH_3$-cyclohex-1-yl | H | H | H | H | $CF_3$ | $CH_3$ | $CH_3$ | 188-190 |
| I.21 | $C(CH_3)_3$ | H | H | Cl | H | H | $C_2H_5$ | $CH_3$ | 158-160 |
| I.22 | 1-$CH_3$-cycloprop-1-yl | H | H | Cl | H | H | $C_2H_5$ | $CH_3$ | 157-158 |
| I.23 | 1-$CH_3$-cyclohex-1-yl | H | H | Cl | H | H | $C_2H_5$ | $CH_3$ | 162-164 |
| I.24 | $C(CH_3)_3$ | H | H | Cl | H | H | $CH_2$-CH=$CH_2$ | $CH_3$ | 112-115 |
| I.25 | 1-$CH_3$-cycloprop-1-yl | H | H | Cl | H | H | $CH_2$-CH=$CH_2$ | $CH_3$ | 90-92 |
| I.26 | 1-$CH_3$-cyclohex-1-yl | H | H | Cl | H | H | $CH_2$-CH=$CH_2$ | $CH_3$ | 115-118 |
| I.27 | $C(CH_3)_3$ | H | H | Cl | H | H | Cyclopropyl | $CH_3$ | 172-175 |
| I.28 | 1-$CH_3$-cycloprop-1-yl | H | H | Cl | H | H | Cyclopropyl | $CH_3$ | 140-142 |
| I.29 | 1-$CH_3$-cyclohex-1-yl | H | H | Cl | H | H | Cyclopropyl | $CH_3$ | 150-152 |
| I.30 | $C(CH_3)_3$ | H | H | Cl | H | H | $CH_3$ | $CF_3$ | |
| I.31 | 1-$CH_3$-cyclohex-1-yl | H | H | Cl | H | H | $CH_3$ | $CF_3$ | |
| I.32 | $C(CH_3)_3$ | H | H | H | H | F | $CH_3$ | $CH_3$ | 178-180 |
| I.33 | 1-$CH_3$-cycloprop-1-yl | H | H | H | H | F | $CH_3$ | $CH_3$ | 124-127 |

| Nr. | $R^1$ | $R^2$ | $R^a$ | $R^c$ | $R^b$ | $R^d$ | $R^3$ | $R^4$ | Fp. [°C] |
|---|---|---|---|---|---|---|---|---|---|
| I.34 | 1-$CH_3$-cyclohex-1-yl | H | H | H | H | F | $CH_3$ | $CH_3$ | 182-184 |
| I.35 | 1-$CH_3$-cyclohex-1-yl | H | H | Cl | H | H | $CH_3$ | $C_2H_5$ | |
| I.36 | $CH(C_2H_5)_2$ | H | H | H | H | Cl | $CH_3$ | $CH_3$ | 144-146 |
| I.37 | $C(CH_3)_3$ | H | H | Cl | H | Cl | $CH_3$ | $CH_3$ | 170-173 |
| I.38 | 1-$CH_3$-cyclohex-1-yl | H | H | Cl | H | Cl | $CH_3$ | $CH_3$ | 140-144 |
| I.39 | $C(CH_3)_3$ | H | H | Cl | H | H | $CH_3$ | H | 142-146 |
| I.40 | 1-$CH_3$-cycloprop-1-yl | H | H | Cl | H | H | $CH_3$ | H | 104-107 |
| I.41 | 1-$CH_3$-cyclohex-1-yl | H | H | Cl | H | H | $CH_3$ | H | Öl |
| I.42 | $C(CH_3)_3$ | H | H | H | H | CN | $CH_3$ | $CH_3$ | 115-119 |
| I.43 | 1-$CH_3$-cycloprop-1-yl | H | H | H | H | CN | $CH_3$ | $CH_3$ | 113-117 |

Analog zu Vorstufe b) des Herstellungsbeispiels wurden die neuen p-Amino-acetanilide III (mit Y = Sauerstoff) der folgenden Tabelle 3 hergestellt:

Tabelle 3

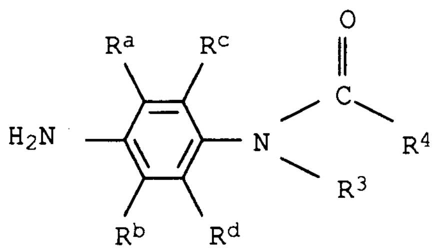

III (Y=O)

| Nr. | $R^a$ | $R^c$ | $R^b$ | $R^d$ | $R^3$ | $R^4$ | Fp. [°C] |
|---|---|---|---|---|---|---|---|
| III.01 | H | Cl | H | H | $CH_3$ | $CH_3$ | Öl |
| III.02 | H | Cl | H | H | $C_2H_5$ | $CH_3$ | 98-99 |
| III.03 | H | Cl | H | H | Cyclo-propyl | $CH_3$ | Öl |
| III.04 | H | Cl | H | H | $CH_2$-CH=$CH_2$ | $CH_3$ | Öl |
| III.05 | H | H | H | $CH_3$ | $CH_3$ | $CH_3$ | Öl |
| III.06 | H | H | H | $CF_3$ | $CH_3$ | $CH_3$ | Öl |
| III.07 | H | F | H | H | $CH_3$ | $CH_3$ | 100-105 |
| III.08 | H | Cl | H | Cl | $CH_3$ | $CH_3$ | Öl |
| III.09 | H | Cl | H | H | $CH_3$ | $C_2H_5$ | Öl |
| III.10 | H | Cl | H | H | $CH_3$ | $CF_3$ | |
| III.11 | H | Cl | H | H | $CH_3$ | $CH_2Cl$ | |
| III.12 | H | Cl | H | H | $CH_3$ | $CH(CH_3)Cl$ | |
| III.13 | H | Cl | H | H | $CH_3$ | $CHCl_2$ | |
| III.14 | H | H | H | CN | $CH_3$ | $CH_3$ | Öl |
| III.15 | H | H | H | Cl | $CH_3$ | H | Öl |

Die folgende Tabelle 4 enthält Nitroaniline V, die analog Rec. trac. Chim. P.B. 51, 878 (1932) hergestellt wurden:

Tabelle 4

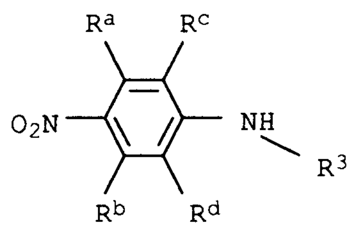

V

| Nr. | $R^a$ | $R^c$ | $R^b$ | $R^d$ | $R^3$ | Fp. [°C] |
|---|---|---|---|---|---|---|
| V.1 | H | Cl | H | H | $CH_3$ | 116-118 |
| V.2 | H | Cl | H | H | $C_2H_5$ | 62-64 |
| V.3 | H | Cl | H | H | Cyclopropyl | 84-86 |
| V.4 | H | Cl | H | H | $CH_2-CH=CH_2$ | 51-54 |
| V.5 | H | H | H | $CH_3$ | $CH_3$ | 138-139 |
| V.6 | H | H | H | $CF_3$ | $CH_3$ | 111-114 |
| V.7 | H | F | H | H | $CH_3$ | 110-114 |
| V.8 | H | H | H | CN | $CH_3$ | 174 (Zers.) |
| V.9 | H | Cl | H | Cl | $CH_3$ | 72-74 |

Beispiel 2

Beispiel für die Überführung eines Nitroanilins V in das entsprechende Anion und dessen anschließender Acylierung:

2-Trifluormethyl-4-nitro-N-methylacetanilid (Tabelle 5, Nr. VI.06)

Zu einer Lösung von 5,50 g (0,025 mol) 2-Trifluormethyl-4-nitro-N-methylanilin in 25 ml Dimethylformamid wurden bei (-5) bis (-10) °C 2,94 g (0,026 mol) Kalium-tert.-butylat gegeben, wonach man noch 30 Minuten bei ca. (-5) °C rührte. Zu der erhaltenen Mischung wurden bei (-5) bis 0 °C unter Rühren 2,80 g (0,027 mol) Acetanhydrid getropft. Anschließend ließ man das Reaktionsgemisch auf etwa 20-25 °C erwärmen und versetzte es nach ca. 15 Stunden mit 250 ml Wasser. Danach extrahierte man dreimal mit je 70 ml Essigester. Die vereinigten organischen Phasen wurden schließlich getrocknet und eingeengt. Ausbeute: 5,60 g (85 %).

Analog zu diesem Beispiel oder zur Vorstufe a) des obigen Herstellungsbeispiels 1 wurden die 4-Nitroacetanilide VI (mit Y = Sauerstoff) der folgenden Tabelle 5 hergestellt:

Tabelle 5

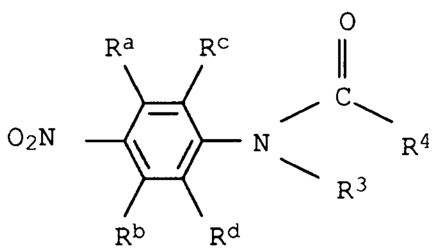

V (Y=O)

| Nr. | $R^a$ | $R^c$ | $R^b$ | $R^d$ | $R^3$ | $R^4$ | Fp. [°C] |
|---|---|---|---|---|---|---|---|
| V.01 | H | Cl | H | H | $CH_3$ | $CH_3$ | 84-86 |
| V.02 | H | Cl | H | H | $C_2H_5$ | $CH_3$ | 113-115 |
| V.03 | H | Cl | H | H | Cyclopropyl | $CH_3$ | 110-112 |
| V.04 | H | Cl | H | H | $CH_2-CH=CH_2$ | $CH_3$ | 67-69 |
| V.05 | H | H | H | $CH_3$ | $CH_3$ | $CH_3$ | 94-95 |
| V.06 | H | H | H | $CF_3$ | $CH_3$ | $CH_3$ | 86-88 |
| V.07 | H | F | H | H | $CH_3$ | $CH_3$ | 94-96 |
| V.08 | H | Cl | H | Cl | $CH_3$ | $CH_3$ | 85-86 |
| V.09 | H | Cl | H | H | $CH_3$ | $C_2H_5$ | |
| V.10 | H | Cl | H | H | $CH_3$ | $CF_3$ | |
| V.11 | H | Cl | H | H | $CH_3$ | $CH_2Cl$ | |
| V.12 | H | Cl | H | H | $CH_3$ | $CH(CH_3)Cl$ | |
| V.13 | H | Cl | H | H | $CH_3$ | $CHCl_2$ | |
| V.14 | H | H | H | CN | $CH_3$ | $CH_3$ | 120-123 |
| V.15 | H | H | H | Cl | $CH_3$ | H | 73-75 |

Die p-Phenylendiamin-Derivate I zeichnen sich durch eine hervorragende Wirksamkeit gegen ein breites Spektrum von pflanzenpathogenen Pilzen, insbesondere aus der Klasse der Phycomyceten, aus. Sie sind z.T. systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sich die Verbindungen I zur Bekämpfung folgender Pflanzenkrankheiten:

* Erysiphe graminis (echter Mehltau) in Getreide,
* Erysiphe cichoracearum und Sphaerotheca fuliginea an Kürbisgewächsen,
* Podosphaera leucotricha an Äpfeln,
* Uncinula necator an Reben,
* Puccinia-Arten an Getreide,
* Rhizoctonia-Arten an Baumwolle und Rasen,
* Ustilago-Arten an Getreide und Zuckerrohr,
* Venturia inaequalis (Schorf) an Äpfeln,
* Helminthosporium-Arten an Getreide,
* Septoria nodorum an Weizen,
* Botrytis cinerea (Grauschimmel) an Erdbeeren, Reben,
* Cercospora arachidicola an Erdnüssen,

* Pseudocercosporella herpotrichoides an Weizen, Gerste,
* Pyricularia oryzae an Reis,
* Phytophthora infestans an Kartoffeln und Tomaten,
* Fusarium- und Verticillium-Arten an verschiedenen Pflanzen,
* Plasmopara viticola an Reben,
* Alternaria-Arten an Gemüse und Obst.

Die neuen Verbindungen können auch im Materialschutz (Holzschutz) eingesetzt werden, z.B. gegen Paecilomyces variotii.

Sie können in die üblichen Formulierungen übergeführt werden, wie Lösungen, Emulsionen, Suspensionen, Stäube, Pulver, Pasten oder Granulate. Die Anwendungformen richten sich dabei nach dem jeweiligen Verwendungszweck; sie sollten in jedem Fall möglichst die feinste Verteilung der Wirkstoffe gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Als Hilfsstoffe kommen dafür im wesentlichen in Betracht:

- Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser;
- Trägerstoffe wie natürliche Gesteinsmehle (z.B. Kaoline, Ton-erden, Talkum, Kreide) und synthetische Gesteinsmehle (z.B. hochdisperse Kieselsäure, Silikate);
- Emulgiermittel wie nichtionogene und anionische Emulgatoren (z.B. Polyoxyethylen-Fettalkohol-Ether, Alkylsulfonate und Arylsulfonate) und
- Dispergiermittel wie Ligninsulfit-Ablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die fungiziden Mittel enthalten im allgemeinen zwischen 0,1 und 95, vorzugsweise zwischen 0,5 und 90 Gew.% Wirkstoff.

Beispiele für solche Zubereitungen sind:

I. eine Lösung aus 90 Gew.-Teilen der Verbindung Nr. I.01 und 10 Gew.-Teilen N-Methyl-α-pyrrolidon, die zur Anwendung in Form kleinster Tropfen geeignet ist;

II. eine Mischung aus 20 Gew.-Teilen der Verbindung Nr. I.02, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes und 40 Mol Ethylenoxid an 1 Mol Ricinusöl; durch feines Verteilen der L°sung in Wasser erhält man eine Dispersion.

III. eine wäßrige Dispersion aus 20 Gew.-Teilen der Verbindung Nr. I.03, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. I.04, 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Nr. I.06, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Nr. I.11 und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. I.28, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Nr. I.24, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. I.38, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Verbindungen I, allein oder in Kombination mit Herbiziden oder Fungiziden, können auch noch mit weiteren Pflanzenschutzmitteln gemischt gemeinsam ausgebracht werden, beispielsweise mit Wachstumsregulatoren oder mit Mitteln zur Bekämpfung von Schädlingen oder Bakterien. Von Interesse ist ferner die Mischbarkeit mit Düngemitteln oder mit Mineralsalzlösungen, welche zur Behebung von Ernährungs- und Spurenelementmängeln eingesetzt werden.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;
Nitroderivate, wie Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthalsäure-diisopropylester;
heterocyclische Substanzen, wie 2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon, 2-Thio-1,3-dithiolo[4,5-b]chinoxalin,
1-(Butylcarbamoyl)-2-benzimidazol-carbaminsäuremethylester,
2-Methoxycarbonylamino-benzimidazol,
2-(Furyl-(2))-benzimidazol,

2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilid,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethyl-morpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2-4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiel

Wirksamkeit gegen Phytophthora infestans an Tomaten Blätter von Topfpflanzen der Sorte "Große Fleischtomate" wurden mit wässriger Spritzbrühe, die 80 Gew.-% Wirkstoff und 20 Gew.-% Emulgiermittel in der Trockensubstanz enthielt, besprüht. Nach 8 Tagen infizierte man die Blätter mit einer Zoosporenaufschwemmung des Pilzes Phytophthora infestans. Die Pflanzen wurden dann in einer wasserdampfgesättigten Kammer bei Temperaturen zwischen 16 und 18 °C aufgestellt. Nach weiteren 6 Tagen hatte sich die Krankheit auf den unbehandelten, jedoch infizierten Kontrollpflanzen so stark entwickelt, daß die fungizide Wirksamkeit der getesteten Verbindungen beurteilt werden konnte.

Gegenüber dem Kontrollversuch (keine Behandlung, 70 % Pilzbefall) zeigte sich, daß die mit den Wirkstoffen Nr. I.01 und I.02 behandelten Pflanzen nur einen Pilzbefall von 0-5 % hatten.

**Patentansprüche**

**1.** p-Phenylendiamin-Derivate der Formel I

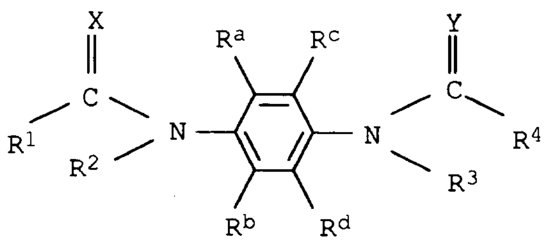

I

in der die Substituenten folgende Bedeutung haben:

| | |
|---|---|
| $R^1$ | $C_3$-$C_8$-Alkyl, $C_2$-$C_8$-Alkenyl, $C_2$-$C_8$-Alkinyl, $C_3$-$C_6$-Cycloalkyl, $C_5$-$C_8$-Bicycloalkyl, $C_5$-$C_8$-Bicycloalkenyl, Dihydropyranyl oder Tetrahydropyranyl, wobei diese Gruppen durch $C_1$-$C_4$-Alkyl, Halogen, $C_1$-$C_4$-Alkoxy, $C_1$-$C_4$-Alkylthio, Cyano, Isocyano, ($C_1$-$C_4$-Alkoxy)-carbonyl, ($C_1$-$C_4$-Alkyl)carbonyl, Azido oder Nitro substituiert sein können; |
| $R^2$ | Wasserstoff oder gemeinsam mit dem $R^1$-CX-N- Teil einen Azetidin-2-on Ring, der gewünschtenfalls ein oder zwei $C_1$-$C_4$-Alkylreste tragen kann; |
| $R^3$ | $C_1$-$C_6$-Alkyl, $C_2$-$C_6$-Alkenyl, $C_3$-$C_6$-Alkinyl, $C_3$-$C_6$-Cycloalkyl, $C_3$-$C_6$-Cycloalkyl-$C_1$-$C_3$-alkyl, $C_1$-$C_4$-Alkoxy-$C_2$-$C_4$-alkyl, $C_3$-$C_4$-Alkenyloxy-$C_2$-$C_4$-alkyl; |
| $R^4$ | Wasserstoff oder $C_1$-$C_6$-Alkyl, $C_2$-$C_6$-Alkenyl, $C_3$-$C_6$-Alkinyl, $C_3$-$C_6$-Cycloalkyl, durch $C_1$-$C_3$-Alkyl substituiertes $C_3$-$C_6$-Cycloalkyl, wobei diese Gruppen jeweils 1 bis 3 Halogenatome tragen können. |
| $R^a$, $R^b$ | Wasserstoff oder Fluor; |
| $R^c$, $R^d$ | Wasserstoff, Cyano, Nitro, Halogen, $C_1$-$C_4$-Alkyl, $C_1$-$C_4$-Alkoxy, $C_1$-$C_4$-Halogenalkyl, ($C_1$-$C_4$-Alkoxy)carbonyl, $C_1$-$C_4$-Alkylcarbonyl oder Formyl, mit der Maßgabe, daß $R^c$ und $R^d$ nicht gleichzeitig Wasserstoff sein können; |
| X, Y | Sauerstoff oder Schwefel. |

**2.** Verwendung der p-Phenylendiamin-Derivate I gemäß Anspruch I als Fungizide.

**3.** Fungizides Mittel, enthaltend flüssige oder feste Trägerstoffe und eine fungizid wirksame Menge mindestens eines p-Phenylendiamin-Derivates der Formel I gemäß Anspruch 1.

**4.** Verfahren zur Bekämpfung von Pilzen, dadurch gekennzeichnet, daß man eine fungizid wirksame Menge mindestens eines p-Phenylendiamin-DerivatesI gemäß Anspruch 1 auf Pilze, vom Pilzbefall bedrohte Pflanzen, deren Lebensraum oder auf das Saatgut der bedrohten Pflanzen einwirken läßt.

**5.** p-Amino-acetanilide der Formel III

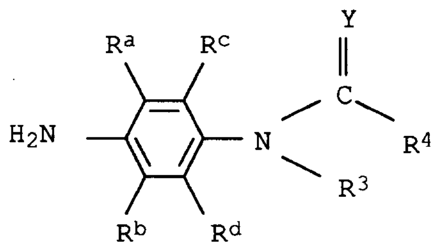

III

in der die Substituenten folgende Bedeutung haben:

- $R^3$ — $C_1$-$C_6$-Alkyl, $C_2$-$C_6$-Alkenyl, $C_3$-$C_6$-Alkinyl, $C_3$-$C_6$-Cycloalkyl, $C_3$-$C_6$-Cycloalkyl-$C_1$-$C_3$-alkyl, $C_1$-$C_4$-Alkoxy-$C_2$-$C_4$-alkyl, $C_3$-$C_4$-Alkenyloxy-$C_2$-$C_4$-alkyl;
- $R^4$ — Wasserstoff, $C_1$-$C_6$-Alkyl, $C_2$-$C_6$-Alkenyl, $C_3$-$C_6$-Alkinyl, $C_3$-$C_6$-Cycloalkyl, durch $C_1$-$C_3$-Alkyl substituiertes $C_3$-$C_6$-Cycloalkyl, wobei diese Gruppen jeweils 1 - 3 Halogenatome tragen können.
- $R^a$, $R^b$ — Wasserstoff oder Fluor;
- $R^c$, $R^d$ — Wasserstoff, Cyano, Nitro, Halogen, $C_1$-$C_4$-Alkyl, $C_1$-$C_4$-Alkoxy, $C_1$-$C_4$-Halogenalkyl, ($C_1$-$C_4$-Alkoxy)carbonyl, $C_1$-$C_4$-Alkylcarbonyl, oder Formyl, mit der Maßgabe, daß $R^c$ und $R^d$ nicht gleichzeitig Wasserstoff sein können;
- Y — Sauerstoff oder Schwefel.

Europäisches Patentamt

# EUROPÄISCHER RECHERCHENBERICHT

Nummer der Anmeldung
EP 95 10 0595

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | EP-A-0 127 990 (SUMITOMO CHEMICAL CO) * Seite 5 - Seite 6; Ansprüche 1,3,4,9,10,12 * | 1-5 | C07C233/42 C07C233/43 C07C233/44 C07D309/08 C07D309/28 C07C327/38 A01N37/22 |
| X | FR-A-1 588 718 (CIBA SOCIÉTÉ ANONYME) * Seite 18 - Seite 20 * | 1-5 | |
| X | FR-A-2 377 999 (SHELL INT RESEARCH) * Ansprüche 1,18 * | 1,5 | |
| D | & BE-A-863 074 | | |
| A | EP-A-0 339 418 (BAYER AG) * Zusammenfassung * | 1,2 | |

RECHERCHIERTE SACHGEBIETE (Int.Cl.6)
C07C
C07D
A01N

Der vorliegende Recherchenbericht wurde für alle Patentansprüche erstellt

| Recherchenort | Abschlußdatum der Recherche | Prüfer |
|---|---|---|
| BERLIN | 24.April 1995 | Rufet, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument